(19) Europäisches Patentamt
European Patent Office
Office européen des brevets

(11) **EP 4 293 121 A2**

(12) **EUROPEAN PATENT APPLICATION**

(43) Date of publication:
**20.12.2023 Bulletin 2023/51**

(21) Application number: **23020290.5**

(22) Date of filing: **14.06.2023**

(51) International Patent Classification (IPC):
**C12P 19/02** (2006.01)    **C07H 3/06** (2006.01)
**C08H 8/00** (2010.01)    **C12P 19/04** (2006.01)
**C12P 19/14** (2006.01)

(52) Cooperative Patent Classification (CPC):
**C12P 19/14; C07H 3/06; C08H 8/00; C12P 19/02; C12P 19/04; C12Y 302/01008**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.06.2022 PT 2022118045**

(71) Applicants:
• **RAIZ - Instituto De Investigação Da Floresta E Papel**
**3800-783 Eixo, Aveiro (PT)**
• **Instituto Superior Técnico**
**1049-001 Lisboa (PT)**

(72) Inventors:
• **De Figueiredo Brites Alves, Ana Maria**
**1049-001 Lisboa (PT)**
• **Dos Santos Serrano, Maria de Lurdes**
**1049-001 Lisboa (PT)**
• **Antunes Henriques, Patrícia Isabel**
**1049-001 Lisboa (PT)**
• **Mendes de Sousa, Antonio Paulo**
**3800-783 Eixo (PT)**
• **De Sousa Pereira, Susana Raquel**
**3800-783 Eixo (PT)**
• **De Oliveira Rodrigues Pinto, Paula Cristina**
**3800-783 Eixo (PT)**
• **Da Silva Almeida, Nazaré**
**3800-783 Eixo (PT)**

(54) **PRODUCTION PROCESS OF XYLOOLIGOSACCHARIDES FROM BLEACHED KRAFT CELLULOSE PULP BY ENZYMATIC HYDROLYSIS IN AN AQUEOUS MEDIUM**

(57) The present invention relates to a process for producing xylooligosaccharides comprising the steps of enzymatic hydrolysis of a bleached Kraft cellulose pulp with an endoxylanase, in an aqueous medium and in the absence of a buffer solution. The separation of the aqueous solution of xylooligosaccharides from the resulting post-hydrolysis cellulose pulp and the purification of the obtained aqueous solution of xylooligosaccharides takes place sequentially.

Another aspect of the present invention concerns the xylooligosaccharides obtained from the described process.

The process makes it possible to avoid any prior treatment of the substrate and the use of buffer solutions to control the pH, while allowing yields similar to, or even superior to, those obtained in conventional processes using buffer solutions. The xylooligosaccharides obtained by the process are those with the main prebiotic activity, with a majority composition of xylobiose and xylotriose.

In addition, the post-hydrolysis cellulose Kraft pulp can be returned to a pulp and paper production process, without causing any decrease in the paper properties of the products produced.

Fig. 1

EP 4 293 121 A2

**Description**

FIELD OF THE INVENTION

The present invention relates to the field of producing xylooligosaccharides, namely a process for the production of xylooligosaccharides by enzymatic hydrolysis of bleached Kraft cellulose pulp in an aqueous medium.

BACKGROUND OF THE INVENTION

The relationship established between the gut microbiome and the immune system, combined with the growing awareness of the need to adopt healthy nutrition, has led, in recent decades, to a greater interest in functional foods, including prebiotics (Mano et al., 2018).

Xylooligosaccharides (XOS) are oligomers derived from xylan, composed of xylose units (X1), with great prebiotic potential (Samanta, Jayapal, Jayaram, et al., 2015). On the regular intake of these non-digestible food compounds, which stimulate the growth and function of intestinal microorganisms, several benefits have been reported, including: maintenance of gastrointestinal health, reduction of blood cholesterol, improvement of body weight, increased absorption of minerals, immune stimulation, glucose-reducing capacity, antioxidant and anticancer activity (Mano et al., 2018; Palaniappan et al., 2021) .

XOS with lower degrees of polymerization (DP), such as xylobiose (X2) and xylotriose (X3), are known to have the strongest prebiotic effect (Gullón et al., 2011; Moura et al., 2007), as well as a relative sweetness similar to that of sucrose (Park et al., 2017), which increases their range of food applications. Regardless of the production process, the final mixture is made up of other compounds that can be seen as impurities and need to be minimized as they affect the functionality of the XOS (Hong et al., 2019). Xylose monomer, for example, although not toxic or carcinogenic at dose levels equal to or less than 2.5% (EFSA, 2018), is absorbed by the body unlike XOS. This absorption is undesirable both due to its caloric and cariogenic characteristics, as well as its unsuitability for consumption by diabetic people (Huntley & Patience, 2018). Due to the importance of these certified prebiotics (EFSA, 2018; FDA, 2013, 2019), their production from various types of lignocellulose biomass has been studied, including softwood and hardwood (Santibáñez et al., 2021). The presence of lignin and cellulose in these substrates can, however, inhibit the hydrolysis of xylan or lead to the formation of unwanted derivative compounds (Hong et al., 2019).

XOS can be produced from xylan present in lignocellulose biomass by enzymatic or chemical methods. The latter includes autohydrolysis and acid hydrolysis (AH) which require high temperatures and pressures, corrosion resistant materials (AH), high energy consumption and often result in the production of high amounts of unwanted by-products (Palaniappan et al., 2021; Samanta, Jayapal, Jayaram, et al., 2015).

Enzymatic hydrolysis stands as the greener alternative for producing XOS. It has been, in the recent past years, under the scope of the food industry due to not requiring any special equipment (low temperatures and pressure), or strong chemical compounds, in contrast to the other common methods (Palaniappan et al., 2021; Santibáñez et al., 2021). Currently, there are certified and commercialized XOS produced by this process (EFSA, 2018).

The careful choice of the most adequate enzyme turns enzymatic hydrolysis into a much more selective process, focusing only on the production of XOS, while minimizing the formation of unwanted by-products, the main products usually being X2 and X3 (Cebin et al., 2021; Palaniappan et al., 2017; Samanta, Jayapal, Kolte, et al., 2015). To assure the enzyme best performance, the control of the solution pH is known to be a very important parameter (Pollet et al., 2010). For this reason, buffer solutions are recurrently used to maintain the activity and stability of the enzymes, improve the reaction yield and reduce enzyme consumption (Palaniappan et al., 2021). The production of XOS in two steps is also documented, i.e., with a pre-treatment of the biomass before the following enzymatic hydrolysis step (Santibáñez et al., 2021).

Patent application KR20190014407A describes an XOS and dissolved pulp production process that involves the steps of adding enzymes such as cellulases and xylanases to a bleached Kraft pulp and subsequent enzymatic reaction, in which the pH of the bleached Kraft pulp is adjusted to around 5.5 using an aqueous hydrochloric acid solution or an aqueous sodium hydroxide solution.

The text of the patent EP1304412B1 discloses a process for producing XOS which consists of the enzymatic reaction of a cellulose pulp, preferably bleached Kraft pulp, occurring its enzymatic reaction with enzymes such as hemicellulases, maintaining a pH control of this enzymatic reaction to an optimum pH between 3 and 10, adding for that an acid or alkaline solution to the reaction system.

Patent application US6942754B2 refers to a process for producing XOS from lignocellulose pulp, which also involves the enzymatic treatment of this pulp with an enzyme, a hemicellulase. The pH of the enzymatic reaction is also adjusted to a value between 3 and 10 by adding an acidic or alkaline solution to the reaction medium.

Despite all the advantages attributed to these enzymatic processes, namely those of being environmentally friendly; having low energy consumptions; moderate operating conditions; high selectivity and low production of unwanted by-products, the large amount of buffer solution and high costs of the enzyme can be, however, major drawbacks for its industrial implementation (Santibáñez et al., 2021). The buffer use in large scale applications increases both the reagents and equipment costs as more complex downstream purification processes are required.

SUMMARY OF THE INVENTION

The present invention relates to a process for producing xylooligosaccharides comprising the following steps:

a) enzymatic hydrolysis of a bleached Kraft cellulose pulp with an endoxylanase, in water or in an aqueous medium, in the absence of a buffer solution, at temperatures between 40 °C and 85 °C and for a period of time of up to 8 hours;
b) separating the aqueous solution of xylooligosaccharides from the post-hydrolysis cellulose pulp resulting from step a);
c) purifying the aqueous solution of xylooligosaccharides obtained in step b).

According to a preferred embodiment, the enzymatic hydrolysis of step a) is carried out at temperatures between 60 °C and 85 °C and for a period of time between 4 and 8 hours.
According to a preferred embodiment, the enzymatic hydrolysis of step a) takes place at temperatures between 75°C and 85°C and for a period of time of 6 hours.
According to a preferred embodiment, the bleached Kraft cellulose pulp is selected from the group consisting of hardwood, softwood and mixtures thereof.
According to a preferred embodiment, the bleached Kraft cellulose pulp is from *Eucalyptus globulus.*
According to a preferred embodiment, the aqueous medium of step a) is a filtrate accompanying a bleached Kraft cellulose pulp.
According to a preferred embodiment, the step c) of purifying the aqueous solution of xylooligosaccharides is selected from the group consisting of extraction and precipitation with solvents, adsorption on activated carbon, vacuum filtration and nanofiltration.
A second aspect of the present invention relates to the xylooligosaccharides obtainable by the process for producing xylooligosaccharides.
According to a preferred embodiment, the xylooligosaccharides comprise at least 50% w/w of xylobiose and 40% w/w of xylotriose.
According to a preferred embodiment, the xxylooligosaccharides comprise at least 0.5% w/w of 4-O-methylglucuronic acid.
A third aspect of the present invention relates to the post-hydrolysis cellulose pulp obtainable by the process for producing xylooligosaccharides.
A fourth aspect of the present invention relates to the use of the post-hydrolysis cellulose pulp wherein it is integrated into a Kraft pulp production process in an amount of up to 3% w/w.

BRIEF DESCRIPTION OF THE DRAWINGS

Figure 1. Influence of experimental conditions on XOS yields of enzymatic hydrolysis with water (HEA - full line) and buffer solution (HET - dashed line). fixing: 62.5°C; 60 U/g pulp; 4 hours (HET e HEA) and pH of 4.5 (HET).

Figure 2. pH variation during enzymatic hydrolysis with water (HEA) and buffer solution (HET) for different concentrations of enzyme (CE). CE 1 < CE 2 < CE 3. HEA, CE1 (■ HEA (CE 1)); (● HEA (CE 2)); (▲ HEA (CE 3)); (♦, HET (CE 2).

Figure 3. Comparison of HEA and HET. ● HEA; ▲ HET pH=4,5; ▲ --- pH=5. Figure A - 41,5°C; 60 U/g pulp. Figure B - 50°C; 30 U/ g pulp; Figure C - 62,5 °C; 60 U /g pulp; Figure D - 75°C; 90 U /g pulp; Figure E - 83,5 °C U/ g pulp.

Figure 4 - Yields on XOS (A) and distribution of degrees of polymerization (B and C) as a function of the time of enzymatic hydrolysis with water (HEA, ▲) and buffer solution (HET, ●) for: 80°C; 100 U/g pulp and pH of 3,7 (HET). (B) HET. (C) HEA. On each bar, from top to bottom: X4; X3; X2; X1.

Figure 5: (A) Production of XOS over time during scale-up performed in 20 L, using water without pH control (●, Mxos/mxylan; ▲, mXOS/mBEKP); (B) Distribution of degrees of polymerization (DP) as a function of the time of the enzymatic hydrolysis with water during scale-up: 75°C; 40 U/g pulp, ▨ X1; ▤, X2; Ⅲ, X3; ▢, X4; ■, X5.

Figure 6: (A) Production of XOS over time during scale-up performed on 20 L, using filtrate accompanying the bleached pulp (●, Mxos/mxylan; ▲, mXOS/mBEKP); (B) Distribution of degrees of polymerization as a function of the time of the enzymatic hydrolysis with water during scale-up: 75°C; 40 U/g pulp, ▢, X1; Ⅲ, X2; ■, X3.

Figure 7. Chemical composition of XOS produced from bleached pulp of E. globulus (BEKP) and commercial XOS from Now Foods (XOS Commercial).

Figure 8. Mass percentage of sugars in the sample of XOS produced from bleached eucalyptus Kraft pulp (A) and commercial XOS (B) during gastrointestinal digestion, ■ Initial, Ⅲ Oral, ▤ Gastric, ▢ Intestinal.

Figure 9. Production of lactate and SCFA (acetate, propionate and butyrate) after 48 h of human fecal inoculum growth, in the absence of prebiotics (Control) or in culture medium enriched with XOS-commercial solution or XOS-BEKP (10 g/L). Results are the average of two independent fermentations and triplicate analysis of each sample $\pm$ standard deviation. ■ Lactate, Ⅲ Acetate, ▤ Proprionate, ▢ Butyrate.

Figure 10. Production of $H_2$ and $CO_2$ after 24 h and 48 h of human fecal inoculum growth, in the absence of prebiotics (Control) or enriched with XOS-commercial solution or XOS-BEKP at 10 g/L. Results are the average of two independent fermentations and triplicate analysis of each sample $\pm$ standard deviation (■, 24 h; □, 48 h) .

Figure 11. Papermaking properties of paper sheets produced with normal market pulp (black bars) and paper sheets produced with 97% (w/w) of normal market pulp and 3% (w/w) of pulp obtained by the XOS production method shown (white bars). The lines indicate the minimums and maximums existing in the specification of a market folder. (A) drainability; (B) hand index; (C) Gurley air resistance; (D) burst index; (E) tear index; (F) traction index.

## DETAILED DESCRIPTION OF THE INVENTION

The present invention relates to a process for the production of xylooligosaccharides (XOS), through the enzymatic hydrolysis of a bleached Kraft cellulose pulp that occurs directly and using water or an aqueous medium as reaction medium, as described in this patent application and claim 1, which allows to avoid any prior treatment of the substrate and the use of chemical products, replacing the buffer solution, traditionally used, by water or an aqueous medium as reaction mediums and, thus, without the need to control the pH, while at the same time allowing to obtain similar yields, or even superior, to those obtained with the use of a buffer solution on a variety of substrates. Additionally, and favorably, since the XOS obtained are those with the main prebiotic activity, with a majority composition of xylobiose (X2) and xylotriose (X3).

This process is also ecologically advantageous as it allows a synergy between the growing demand for prebiotics and the pulp and paper industry, based on the production of xylooligosaccharides using the enzymatic hydrolysis of a bleached cellulose Kraft pulp. This intermediate product mainly composed of cellulose and hemicellulose, with 16 - 23% of xylan (Marques et al., 2019; Shatalov et al., 1999), although very little explored for the production of XOS, it is widely produced and available in this industrial sector, representing a low-cost raw material. In addition, post-hydrolysis cellulose Kraft pulp, obtained by the process described here, can be recycled into a pulp and paper production process, without causing any decrease in the paper properties of products of this type produced, thus being able to originate a waste-free XOS production method. Furthermore, by using only water, the downstream purification of the XOS solution is also simplified, as well as any wastewater treatment.

Considering that one of the main arguments against enzymatic hydrolysis has always been the high cost of enzymes and reagents, the fact that it is feasible to produce XOS using water or an aqueous medium as the reaction medium, without the need to add a buffer solution, allows for a less expensive production methodology, being an important improvement for industrial implementations.

This type of compounds, of a polymeric nature, have a composition and intrinsic characteristics that directly depend on the process used for their production and the raw materials involved.

In the context of the present invention, bleached Kraft cellulose pulp relates to cellulose pulp produced through a chemical process that causes the dissolution of wood lignin in order to release the fibers from the matrix, in this case a Kraft process, or sulfate cooking. This chemical process consists of cooking the wood in a cooking liquor usually made up of sodium hydroxide and sodium sulfide, at temperatures of the order of 140 to 180 °C. The pulp is also subjected to a bleaching step, whose purpose is to continue the delignification by the action of oxidizing agents such as, for example, oxygen, chlorine dioxide and hydrogen peroxide.

In the context of the present invention, the filtrate that accompanies a bleached Kraft cellulose pulp, never-before-dried, refers to the filtrate that accompanies the pulp after the last bleaching stage, of a Kraft pulp production process, and consists of an aqueous solution with salt residues from the wood itself and from the bleaching process (sodium, calcium, magnesium, potassium and silica).

In the context of the present invention, post-hydrolysis cellulose pulp refers to the pulp obtained after the XOS production process described herein, which involves the enzymatic hydrolysis of a bleached Kraft cellulose pulp in water or in an aqueous medium.

In the context of the present invention, purification of the aqueous solution of xylooligosaccharides is carried out by processes such as, but not limited to, solvent extraction and precipitation, activated carbon adsorption, vacuum filtration and membrane separation processes.

The xylooligosaccharide production process described here consists of the enzymatic hydrolysis of a bleached Kraft cellulose pulp with an endoxylanase, in water or in an aqueous medium, in the absence of a buffer solution, at temperatures between 40 °C and 85 °C and for a period of time of up to 8 hours, followed by the separation of the aqueous solution of xylooligosaccharides from the resulting post-hydrolysis cellulose pulp and the purification of the aqueous solution of xylooligosaccharides obtained in the process.

Examples

The different analytical methods listed below were considered:

a. Hemicelluloses (Pentosans)

Xylan quantification in the pulp was determined through pentosan content, from which 99% corresponds to xylan (Pinto et al., 2005), using the Seaman hydrolysis method (Selvendran et al., 1979). The pulp was soaked in 400 $\mu$L of 72% (w/w) $H_2SO_4$ at 25°C for 3 hours and then diluted in 4.4 mL of distilled water at 100°C for 2.5 hours. The amount of xylose produced was determined by HPLC and the result multiplied by 0.88 to give the pentosan content.

b. Humidity

The humidity of the pulp was determined, in the course of the experiments, by the methodology described in TAPPI T 412 om-11, 2011.

c. High Performance Liquid Chromatography (HPLC)

Prior to HPLC analysis, all the samples were filtered through a 0.2 $\mu$m syringe filter.

The enzymatic hydrolysis samples were eluted with 0.2 mL/ min mili-Q water at 70°C using an Aminex HPX 42A column from Biorad® and analyzed through a refractive index (RI) detector model 133 Gilson (detection limit $\geq$ 5 ppm).

Considering the HPLC results, the yields of XOS formation were defined by the following equations:

$$Yield\ per\ xylan\ (\%) = \frac{m_{XOS}\ (g)}{m_{xylan\ in\ the\ pulp}\ (g)} \times 100 \qquad (1)$$

$$Yield\ per\ pulp\ (\%) = \frac{m_{XOS}\ (g)}{m_{initial\ dry\ pulp}\ (g)} \times 100 \qquad (2)$$

Where $m_{XOS}$ refers to the mixture of X2 + X3 + X4 +...+ Xn.

The samples resulting from pentosan content determination were eluted with 0.2 mL/ min of $H_2SO_4$ 5 mM at 50°C using an Aminex HPX 87H column from Biorad® and analyzed through a RI detector model 2142 LKB Bromma (detection limit $\geq$ 10 ppm).

A bleached Kraft pulp of Eucalyptus globulus (BEKP) was used, whose main characteristics are presented in Table 1.

Table 1. Characteristics of the used Kraft pulp.

| Intrinsic Viscosity (mg/ L) | Polymeric degree | SR[1] | pH | Ashes (%w/w) | Humidity (%) | Hemicelluloses Content (%) |
|---|---|---|---|---|---|---|
| 960 $\pm$ 12 | 2837 $\pm$ 47 | 30° | 4.06 $\pm$ 0.05 | 0.25 $\pm$ 0.01 | 6.0 $\pm$ 0.3 | 21.4 $\pm$ 0.4 |
| [1] Schopper-Riegler: drainage capacity. | | | | | | |

Standard solutions of XOS with different degrees of polymerization, xylobiose (X2), xylotriose (X3), xylotetraose (X4), xylopentaose (X5) and xylohexaose (X6), were obtained from Megazyme, Ireland. All the other chemicals, including xylose (X1) > 99%, glacial acetic acid ($CH_3COOH$) 100% and sodium acetate ($C_2H_3NaO_2$) 99%, were from Sigma-Aldrich Company, USA.

The enzyme used in the hydrolysis, an endoxylanase, belonging to the GH 11 family (GH - Glycoside Hydrolase), assayed for its enzyme activity according to endo-xylanase activity (Bailey et al., 1992). One unit (U) is defined as the amount of enzyme that liberates 1 $\mu$mol of xylose per minute under the assay conditions.

The reaction medium was first heated in a stirred jacketed reactor (200 mL) and, after reaching the defined temperature, a given amount of endo-xylanase was added and mixed during 2 - 3 minutes. The amount of pulp for a total consistency of 4% was then introduced. At this stage the hydrolysis begins, with the temperature and the stirring (100 rpm) remaining constant and the pH being monitored during all the reaction time. Samples were taken at predefined periods of 1, 2, 4, 6 and 8 hours, heated in boiling water to deactivate the enzyme and posteriorly analyzed by high-performance liquid chromatography (HPLC).

At the end of the reaction, the suspension was submitted to vacuum filtration, to separate the residual pulp from the filtrate containing XOS. The pulp was then dried at 60°C and further analyzed to determine the remaining pentosan content.

Example 1

An enzymatic hydrolysis with water (HEA) was carried out at temperatures of 40 °C, 60 °C and 85 °C and with amounts of 10 - 110 U/g pulp for enzyme concentration, in periods of time between 1 h - 6 h, and according to the process described above. The maximum yields determined from equations 1 and 2 were in the ranges of 20-32 °C.

Example 2

Two different approaches were considered regarding the reaction medium: i) enzymatic hydrolysis with acetate buffer solution (HET) and ii) enzymatic hydrolysis with water (HEA), and according to the procedure described in the "Examples" section. Value ranges varied between 41.5 - 83.5 °C for temperature, 9.5 - 110.5 U/g pulp for enzyme concentration and for the pH of the solution, when buffer solution is used, 3.7 - 5.3.

Tables 2 and 3 represent the conditions and results of the reactions carried out with buffer solution (Table 2) and water (Table 3) and respective yields, determined from equations 1 and 2.

Table 2. Conditions and yields of enzymatic hydrolysis with buffer solution (HET).

| Temperature (°C) | Enzyme Concentration (U/ g pulp) | pH | (a) XOS yield/ Xylan (b) XOS yield / Pulp | | |
|---|---|---|---|---|---|
| | | | 1 - 8h | 4 - 8h | 6h |
| 41.5 | 60.0 | 4.5 | (a) 14-21% (b) 2.5-3.7% | (a) 19-21% (b) 3.3-3.7% | (a) 20% (b) 3.6% |
| 50.0 | 30.0 | 4.0 | (a) 12-18% (b) 2.1-3.2% | (a) 15-18% (b) 2.6-3.2% | (a) 17% (b) 2.9% |
| 50.0 | 30.0 | 5.0 | (a) 12-21% (b) 2.2-3.7% | (a) 18-21% (b) 3.1-3.7% | (a) 19% (b) 3.4% |
| 50.0 | 90.0 | 4.0 | (a) 15-27% (b) 2.7-4.9% | (a) 22-27% (b) 3.9-4.9% | (a) 26% (b) 4.6% |

(continued)

| Temperature (°C) | Enzyme Concentration (U/ g pulp) | pH | (a) XOS yield/ Xylan (b) XOS yield / Pulp | | |
|---|---|---|---|---|---|
| | | | 1 - 8h | 4 - 8h | 6h |
| 50.0 | 90.0 | 5.0 | (a) 15-24% (b) 2.7-4.2% | (a) 21-24% (b) 3.7-4.2% | (a) 23% (b) 4.1% |
| 62.5 | 9.5 | 4.5 | (a) 9-17% (b) 1.7-3.0% | (a) 16-17% (b) 2.8-3.0% | (a) 17% (b) 2.9% |
| 62.5 | 60.0 | 3.7 | (a) 17-29% (b) 3.1-5.2% | (a) 25-29% (b) 4.5-5.2% | (a) 28% (b) 4.9% |
| 62.5 | 60.0 | 4.5 | (a) 15-26% (b) 2.7-4.5% | (a) 20-26% (b) 3.5-4.5% | (a) 25% (b) 4.4% |
| 62.5 | 60.0 | 5.3 | (a) 16-23% (b) 2.8-4.1% | (a) 22-23% (b) 3.9-4.1% | (a) 23% (b) 4.1% |
| 62.5 | 110.5 | 4.5 | (a) 17-27% (b) 3.0-4.7% | (a) 23-27% (b) 4.1-4.7% | (a) 25% (b) 4.5% |
| 75.0 | 30.0 | 4.0 | (a) 15-24% (b) 2.7-4.3% | (a) 22-24% (b) 4.0-4.3% | (a) 24% (b) 4.2% |
| 75.0 | 30.0 | 5.0 | (a) 15-22% (b) 2.7-3.9% | (a) 20-22% (b) 3.6-3.9% | (a) 22% (b) 3.8% |
| 75.0 | 90.0 | 4.0 | (a) 22-33% (b) 4.0-5.9% | (a) 30-33% (b) 5.3-5.9% | (a) 33% (b) 5.9% |
| 75.0 | 90.0 | 5.0 | (a) 18-27% (b) 3.2-4.8% | (a) 25-27% (b) 4.5-4.8% | (a) 27% (b) 4.8% |
| 83.5 | 60.0 | 4.5 | (a) 17-23% (b) 3.0-4.1% | (a) 22-23% (b) 3.9-4.1% | (a) 23% (b) 4.1% |

Table 3. Conditions and yields of enzymatic hydrolysis with water (HEA).

| Temperature (°C) | Enzyme Concentration (U/ g pulp) | (a) XOS yield/ Xylan (b) XOS yield / Pulp | | |
|---|---|---|---|---|
| | | 1 - 8h | 4 - 8h | 6h |
| 41.5 | 60.0 | (a) 10-21% (b) 1.8-3.8% | (a) 19-21% (b) 3.3-3.8% | (a) 20% (b) 3.5% |
| 50.0 | 30.0 | (a) 10-22% (b) 1.7-3.8% | (a) 18-22% (b) 3.2-3.8% | (a) 20% (b) 3.6% |
| 50.0 | 90.0 | (a) 13-25% (b) 2.4-4.4% | (a) 22-25% (b) 3.8-4.4% | (a) 24% (b) 4.2% |
| 62.5 | 9.5 | (a) 8-20% (b) 1.5-3.6% | (a) 17-20% (b) 3.0-3.6% | (a) 19% (b) 3.4% |
| 62.5 | 60.0 | (a) 12-25% (b) 2.2-4.3% | (a) 20-25% (b) 3.5-4.3% | (a) 23% (b) 4.1% |
| 62.5 | 110.5 | (a) 13-26% (b) 2.3-4.5% | (a) 23-26% (b) 4.0-4.5% | (a) 25% (b) 4.4% |

(continued)

| Temperature (°C) | Enzyme Concentration (U/ g pulp) | (a) XOS yield/ Xylan (b) XOS yield / Pulp | | |
|---|---|---|---|---|
| | | 1 - 8h | 4 - 8h | 6h |
| 75.0 | 30.0 | (a) 10-20% (b) 1.8-3.5% | (a) 17-20% (b) 3.0-3.5% | (a) 19% (b) 3.4% |
| 75.0 | 90.0 | (a) 16-32% (b) 2.8-5.6% | (a) 26-32% (b) 4.7-5.6% | (a) 30% (b) 5.3% |
| 83.5 | 60.0 | (a) 13-24% (b) 2.3-4.3% | (a) 20-24% (b) 3.6-4.3% | (a) 22% (b) 4.0% |

The maximum XOS yields obtained per xylan and per pulp, respectively (equations 2 and 3), stood in the range of 17.1 - 33.4% and 3.0 - 5.9% using buffer solution and between 20.0 - 31.7% and 3.5 - 5.6% for using water as reaction medium, as described in tables 2 and 3. The resulting hydrolysates contained mostly xylobiose and xylotriose, compounds known to have a greater prebiotic effect. The formation of xylotetraose was also observed, however, to a lesser extent. Despite the different degrees of polymerization produced, for simplification purposes, they will all be considered together as XOS.

Alongside XOS production, xylose, an unwanted by-product without prebiotic properties, was also formed in small amounts. Besides this, no other reaction product was detected, as it is common for this type of process unlike for acid hydrolysis or autohydrolysis.

At the end of the enzymatic treatment, an average pentosan content between 10 - 14% was determined for the hydrolyzed pulp.

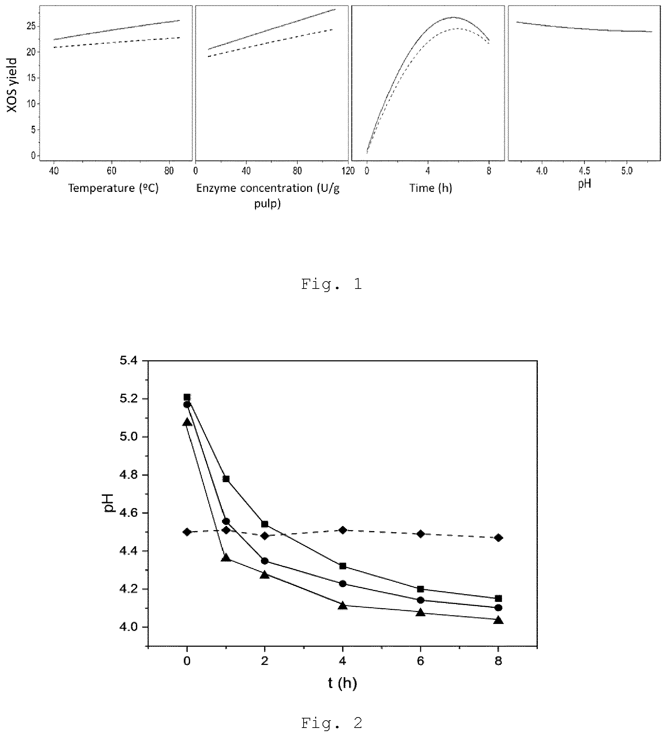

Figure 1 shows the variation of XOS yields as a function of each parameter for the use of buffer solution and water, setting the conditions: 62.5°C; 60 U/g pulp; 4 hours (HET and HEA) and pH 4.5 (HET). It can be seen that the influence of the different parameters is similar, regardless of the reaction medium used. As for the reaction time, the XOS yields increase considerably up to 4 - 5 hours, after which a tendency towards stabilization or, in some cases, a decrease is observed.

Although not as significant, the high temperatures also favored the production of XOS.

Regarding the pH for the HET, its variation has the least effect on the yields. However, lower pH values (< 4.0) seem to result in greater XOS production.

For HEA, as the pH of the solution is not controlled, the influence of this parameter was not considered. Unlike HET, in which the predefined pH remains constant throughout the hydrolysis, for HEA the initial pH value of the water decreases rapidly at the moment the reaction starts, as shown in Figure 2. A sharper decline is observed in the first hours of reaction followed by a stabilization trend. This reduction may be related to the formation of the enzyme-substrate transitional complex, which alters the configuration of the enzyme and releases hydrogenions (H+), resulting in a decrease in the pH of the solution. In this context, the stabilization tendency is therefore related to the enzyme dosage used, as confirmed in Figure 2. The higher the enzyme concentration (EC3) the faster the pH decreases, thus tending towards lower values. However, these low pH values achieved are not problematic, since, for the considered range, this parameter has a low impact on the reaction yields. Furthermore, although with little influence, lower pH values favor the production of XOS, as seen in the HET parametric analysis in Figure 2.

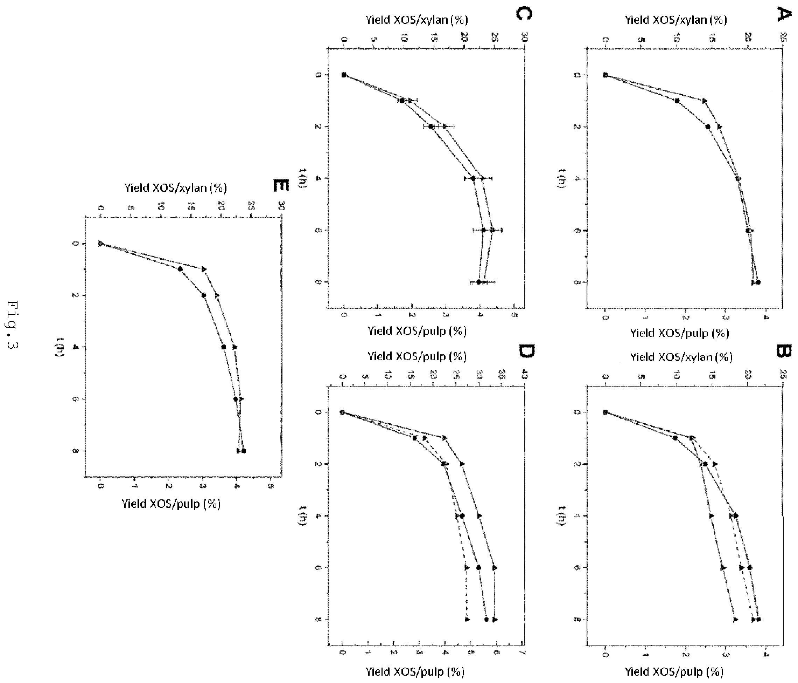

Figure 3 presents, in greater detail, the comparison of enzymatic hydrolysis yields for some examples at different temperatures. It is confirmed, again, that the yields derived from the use of water as the reaction medium are, for the most part, identical to the use of the buffer solution. The greatest difference was observed, however, for higher enzyme concentrations (Figure 4-D), where the influence of pH is more pronounced. In this case, the yield of HEA was similar to HET at pH 5.0 up to 4 hours of reaction, becoming higher after 6 hours. On the other hand, compared to the buffer solution at pH 4.0, the yields were lower, with the greatest difference observed at 6h of reaction, of 3.5% and 0.6% per xylan and per pulp, respectively.

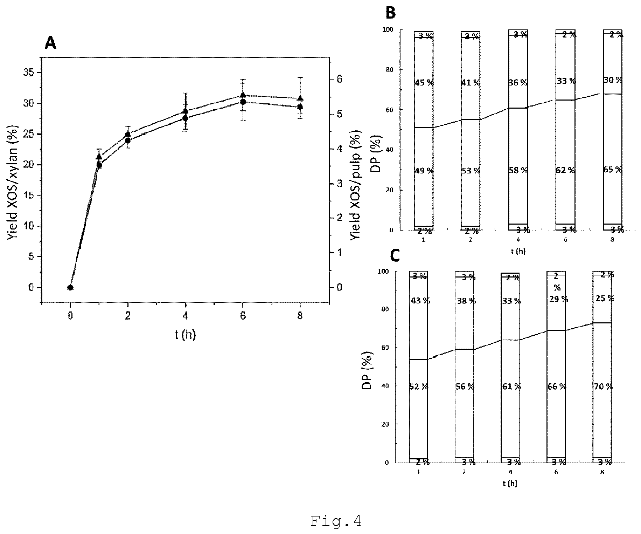

In summary, from this analysis, it could be concluded that the influence of the different reaction parameters studied for the enzymatic production of XOS was similar when using buffer solution or water, leading to operational conditions in which the maximum yield was identical: high temperatures ($\geq$ 75°C) and enzyme concentrations ($\geq$ 90 U/g pulp), low pH values (for HET) and reaction times between 4 and 6 hours. Taking this into account, tests were carried out for both reaction media, for the following conditions: 80 $\pm$ 2°C; 100 $\pm$ 10 U/g pulp and pH 3.7 $\pm$ 1 for HET. As the influence of temperature on the yield is low, a lower temperature value can also be used to reduce energy consumption. The HEA and HET results for the referred conditions are compared in Figure 4.

In Figure 4-A, it is possible to observe that the XOS yield profile throughout the reaction was practically identical for both reaction media. The maximum yields obtained are equivalent for buffer solution and water, these being $30.3 \pm 3.0\%$ and $31.4 \pm 2.6\%$, respectively, both at 6 hours. The distribution of degrees of polymerization (Figure 4 - B, C) was also similar for the analyzed situations, with a gradual decrease in the amount of xylotriose as the reaction proceeded and, consequently, an increase in the formation of xylobiose. The main difference between HET and HEA reactions was found in the distribution of xylobiose and xylotriose. Although the amount of X2 was always higher than that of X3 for both reaction media, there was a slightly higher production of X3 when buffer solution was used. For the maximum value of yield (6h), the composition of the enzymatic hydrolyzate was 62% in X2 and 33% in X3 when buffer solution was used and 66% in X2 and 29% in X3 for water.

As noted earlier, buffer solutions are generally necessary to preserve enzyme stability and activity. Its use at an industrial level is, however, associated with a significant increase in production costs. Thus, the use of an alternative reaction medium such as water, instead of a buffer solution, is an important process improvement both from an economic and environmental point of view.

The viability of replacing the buffer solution, frequently used in enzymatic hydrolysis, with water and without losing important products has been proven. In addition, the results always show a higher amount of xylobiose in the reaction mixture, which is an additional advantage since it is the oligosaccharide with the greatest prebiotic effect. The enzymatic hydrolysis was thus carried out directly on the bleached Kraft pulp of Eucalyptus globulus using water, without the need for any pre-treatment of the biomass, such as, for example, an alkaline or acid treatment, resulting in more than 5 g of XOS/100 g of dry pulp (Figure 4).

Example 3

A scale-up of the enzymatic hydrolysis of about 100x was carried out, with a reaction medium of 20 L. This scale-up was carried out with water (HEA) and also with the filtrate that accompanies the never-before-dried bleached pulp. This filtrate accompanies the pulp after the last bleaching stage and consists of an aqueous solution with salt residues from the wood itself and from the bleaching process (sodium, calcium, magnesium, potassium and silica). In this case, it was only necessary to adjust the pH to 5.0 of the filtrate with the addition of NaOH before starting the enzymatic hydrolysis, given that the initial pH is < 4.0, and during the hydrolysis no pH control was carried out.

The procedure for this scale-up was identical to the one described above. 4 samples were taken over 5 hours for the pulp with water (HEA) test and 7 hours for the pulp and filtrate (HEF) test. Sample treatment and analysis were identical to those described above. Table 4 represents the conditions and results for the yields obtained, determined from equations 1 and 2.

Table 4. Conditions and yields of enzymatic hydrolysis with water (HEA) and with pulp filtrate (HEF), in a 20 L reaction medium.

| Temperature (°C) | Enzyme Concentra tion (U/ g pulp) | Medium | (a) XOS yield/ Xylan (b) XOS yield / Pulp | | |
|---|---|---|---|---|---|
| | | | 1 - 5h | 3 - 5h | 5h |
| 70.0 | 60.0 | Water | (a) 21-29% (b) 4.1-5.6% | (a) 27-29% (b) 5.2-5.6% | (a) 29% (b) 5.6% |
| | | | 1 - 7h | 5 - 7h | 7h |
| 70.0 | 60.0 | Pulp filtrate | (a) 13-31% (b) 2.3-5.5% | (a) 12-31% (b) 2.2-5.5% | (a) 31% (b) 5.5% |

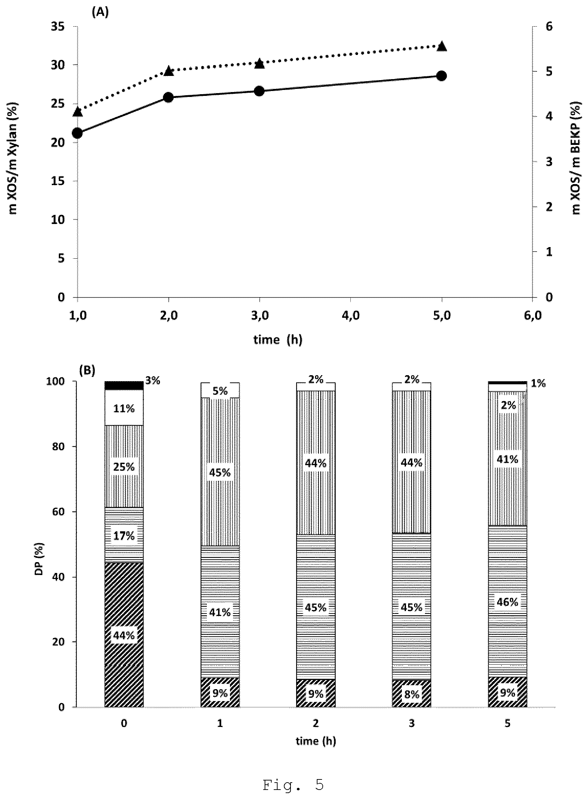

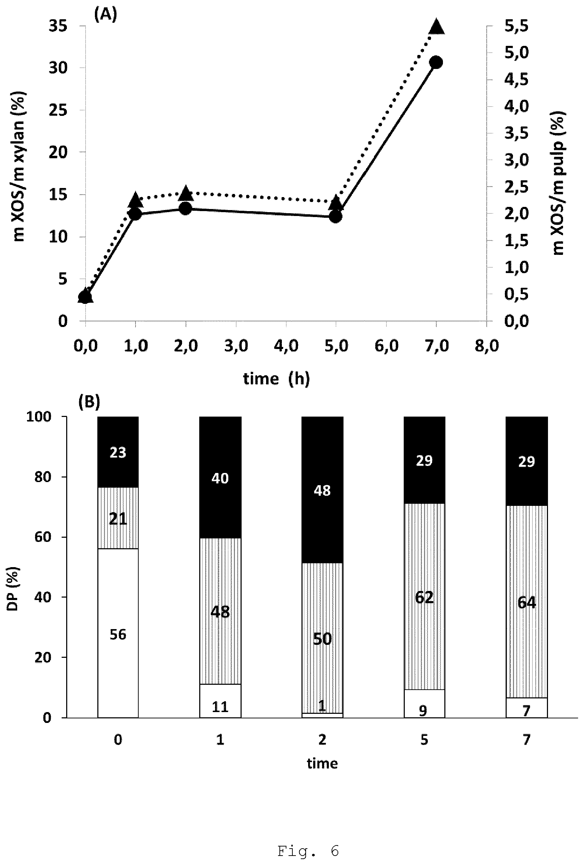

In the case of HEA, by 5 h (Figure 5-A) 28.6% (m XOS/m Xylan) were produced, close to what was produced on a smaller scale (31.4 %), although the amount of enzyme in the 20 L assay was about 60 % below of what was used in the reactions described above (60 instead of 100 U/g). The degrees of polymerization obtained are also in accordance with the previous hydrolysis, with X2 and X3 being the predominant oligosaccharides (46 and 41%, respectively), with a residual of X4 and X5 (3%) existing at 5h (Figure 6-B). In the case of HEF, at 7 am (Figure 6-A) the XOS produced were 30.6% ($w_{XOS}/ w_{Xylan}$). This yield is in line with those obtained both in the case of the HEA 20L and in the case of the smaller scale HEA. This confirms, once again, the success of the enzymatic hydrolysis for the production of XOS using water without pH control and using, in this case, filtrate from the bleached pulp. The degrees of polymerization obtained are also in accordance with the previous reactions, with X2 being the predominant oligosaccharide (64%), followed by X3 (30%) at 7 h (Figure 6-B).

The filtrate obtained after the 20 L HEF, together with the waters from the pulp washings carried out (total of 32 L), was purified in a Nanofiltration (NF) system, using an NF270 polymeric membrane with a cut-off of 270 Da. NF was performed under the following conditions: 16 °C, flow rate of 1.5 L.h$^{-1}$/m$^2$ at a pressure of 3 bar, for 250 min. The two streams obtained at the end of this process (permeate and final concentrate) were frozen for later analysis by HPLC, to determine the XOS concentration, according to the procedure already described in point "c.".

For the analysis of this purification process, some factors were calculated, namely:

$$Mass\ recovery(\%) = \frac{Feed\ concentration\ -\ Permeate\ Concentration}{Feed\ concentration} \times 100 \quad (3)$$

$$Mass\ Concentration\ Factor = \frac{Feed\ concentration}{Final\ concentrate\ concentration} \quad (4)$$

$$Loss\ (\%) = \left(1 - \left(\frac{Final\ permeate\ concentration + Final\ concentrate\ concentration}{Feed\ concentration}\right)\right) \times 100 \quad (5)$$

The final concentrate obtained after NF was lyophilized and extensively characterized, according to the following techniques:

Ashes: method according to standard_TAPPI T 211 om-02.

Glucose and glucose dimers: analysis carried out by high performance anion exchange chromatography with pulsed amperometric detector (HPAEC-PAD) Dionex ICS-5000+ DC, with the CarboPac PA20 column according to the analysis method:

A: H$_2$O B: NaOH 0,2M
Flux: 0.27 ml/ min
T= 30° C
t= 60 min, according to the gradient:
0-15 min (90 A/10 B)
15-30 min (gradual ascent of 90A/10B to 0A/100B)
30-35 min (0A/100B)
35-60 min (90A/10B)

All samples analyzed qualitatively and quantitatively were filtered through a 0.2 $\mu$m syringe filter.
XOS (X>6) - XOS submitted to mild acid hydrolysis (9 % H$_2$SO$_4$, 100 °C for 2.5h) to determine the total xylose content of the sample. % of X>6 calculated based on the difference between the total xylose (after acid hydrolysis) and the theoretically existing xylose taking into account the composition of X1, X2, X3, X4, X5 and X6. Samples after acid hydrolysis analyzed by HPAEC-PAD.
Glucose oligosaccharides: samples subjected to mild acid hydrolysis (9 % H$_2$SO$_4$, 100 °C for 2.5h) to determine the total glucose content of the sample. % glucose >2 calculated based on the difference between the total glucose (after acid hydrolysis) and the theoretically present glucose taking into account the composition of glucose monomer and dimer detected in the sample without acid hydrolysis. Samples after acid hydrolysis analyzed by HPAEC-PAD.
Protein: % N determined by elemental analysis. Protein calculated based on the multiplication of the % N found by the factor 6.25 (Krul, E.S. 2019).
With the NF purification process, the XOS (X2 to X5) were concentrated by an average of 9.0x (Table 5), with xylose (X1) being the sugar whose concentration effect was less noticeable, around 2.0x. This effect is positive, as xylose, being a monosaccharide, will not contribute to the desired prebiotic effects. During this process, an average loss of XOS 37% to the membrane was also observed.

Table 5: Final concentration of streams obtained after nanofiltration and respective concentration factors, rejection and XOS losses (according to equations 3, 4 and 5).

|  | X1 | X2 | X3 | X4 | X5 |
|---|---|---|---|---|---|
| Feed (g/L) | 0,15 | 0,62 | 0,57 | 0,06 | 0,01 |

(continued)

|  | X1 | X2 | X3 | X4 | X5 |
|---|---|---|---|---|---|
| Final permeate (g/L) | 0,08 | 0,21 | 0,08 | 0,01 | 0,00 |
| Final XOS Concentrate (g/L) | 0,31 | 4, 6 | 5,7 | 0,64 | 0,12 |
| Mass recovery (% w/w) | 11 | 40 | 53 | 55 | 50 |
| Mass concentration factor | 2 | 7 | 10 | 10 | 9 |
| Loss(% w/w) | 37 | 28 | 34 | 35 | 50 |

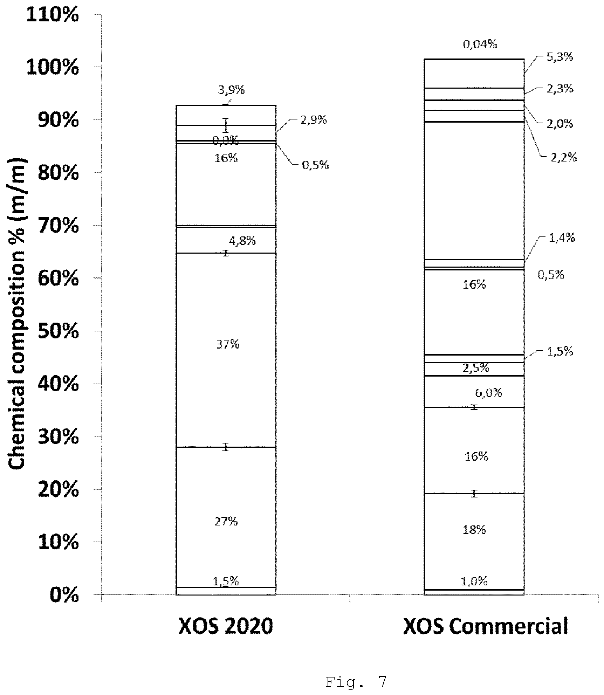

After purification by NF, the reject obtained was lyophilized and characterized (Figure 7). A comparison was also made with commercially available XOS from NOW Foods (XOS-Commercial). It was observed that the XOS obtained by the method of the present invention (XOS BEKP), have a higher XOS content (84% w/w), while the commercial XOS contain only 62% (w/w). Contributing to this fact is the glucose (Glc) and derivatives content of Commercial XOS, around 28% (Glc, Glc dimers and oligosaccharides). It should be noted that in this characterization XOS with a higher degree of polymerization (DP) were detected, namely X4, X5 and X6, possibly due to the observed concentration effect, both during NF and during lyophilization itself. The characterization carried out during and after enzymatic hydrolysis, contemplates aliquots of XOS solution of a total of 20L, naturally XOS with DP > 3 are found in low concentrations and not directly detectable in the suspension, but detectable and quantifiable after the performed purification and drying steps. The XOS obtained by the method of the present invention also contain uronic acid derivatives, namely 4-O-methyl glucuronic acid (4-O-MeGlcA). In the case of commercial XOS, residues of arabinose (Ara) and galactose (Gal) were found. According to the characterization carried out, it is also observed that the amount of protein (from the enzyme) is identical in the XOS obtained by the method of the present invention (XOS BEKP) and in the commercial XOS, 2.9 and 2.3%, respectively.

The XOS produced in the 20 L assay and purified by NF, were characterized to understand the existing biological activity. After lyophilization, the XOS were subjected to a gastrointestinal digestive simulation, comprising the oral, stomach and small intestine phases of the gastric system, with the objective of determining the amount of XOS that effectively reaches the large intestine for metabolization by probiotic bacteria, to prove the effective prebiotic activity. These results were also compared to those of commercially available XOS from NOW Foods (XOS-Commercial).

To digest the samples, the in vitro static method standardized by INFOGEST was used, which simulates the phases: oral, gastric and small intestine (Minekus et al. 2014). In this analysis, the identification of carbohydrate profiles during digestion, an HPLC was used under the following conditions:

1) Shodex column (Shodex Asahipak NH2P-50 4E 100Å 5$\mu$m, 4.6 x 250mm), 30 °C, eluent - acetonitrile:water (68:32) with 0.04% ammonia in water; 1 mL/min volume flow rate, detector - Refractive Index.
2) MetCarb 87H column (Varian, 300 x 7.8 mm), 60 °C, eluent - 0.005 M $H_2SO_4$; 0.6 mL/min volume flow rate, detector - Refractive Index.

An in vitro intestinal model with a human fecal inoculum from a healthy donor was subsequently used to evaluate and compare the prebiotic potential of commercial XOS (XOS-Commercial) and XOS produced from Eucalyptus Kraft pulp (XOS-BEKP, according to the following methodology:

Fecal inoculum: The fecal sample was obtained from a healthy human volunteer who had not taken antibiotics, pre or probiotic supplements for 3 months prior to the study. The 28-year-old male donor does not smoke and follows a Mediterranean diet. The sample was collected on site, diluted in phosphate buffered saline (0.1 M pH 7.0) and maintained anaerobically (N2 100%) at 4°C overnight prior to inoculation.

In vitro fermentations: static fermentations were carried out in a batch regime, at 37 °C, for 48 h in anaerobic culture flasks. XOS solutions were added to the culture medium at a final concentration of 10 g/L. A fecal inoculum concentration of approximately 10% (v/v) was used. Anaerobic conditions were maintained by previously pressurizing the headspace of the flasks with nitrogen to 170 KPa. Liquid samples were collected at 0, 24 and 48 h, centrifuged at 4000 x g for 10 min and the supernatant was subsequently used for HPLC analyses. Headspace gas samples were taken at 24 and 48h and were used to determine gas production. The pH and ammonia concentration were measured after 48 h. The fermentation medium was removed from the bottles after 48h, centrifuged, washed, suspended in phosphate saline buffer (PBS) (0.1 M at pH 7.0) and stored at -20 °C for DNA extraction. Fermentations were performed in duplicate, using a blank, without added prebiotics, as a negative control.

Analytical techniques: The production of lactate and short-chain fatty acids (SCFAs), such as acetate, propionate and butyrate, was evaluated by analysis by HPLC liquid chromatography (Knauer, Berlin, Germany) equipped with a Knauer-RI detector and an Aminex HPX 87H column (300 mm x 7.8; Biorad, Hercules, CA). Gas production was evaluated by gas chromatography (GC), using a Bruker Scion 456-GC (Billerica, MA) equipped with a thermal conductivity detector and two columns: BR-QPLOT and Molsieve. The ammonia concentration was determined with an enzymatic kit (LCK 302) from Hach-Lange (Düsseldorf, Germany).

Microbiota analysis: Total DNA from fecal inoculum and fecal fermentations (48 h) was extracted with the SPIN FastDNA Soil Kit (MP Biomedicals, Solon, OH) according to the manufacturer's instructions. High-throughput sequencing (16S rRNA gene) by Illumina MiSeq technology was performed by RTL Genomics (Lubbock, TX, USA), and samples were analyzed in duplicate.

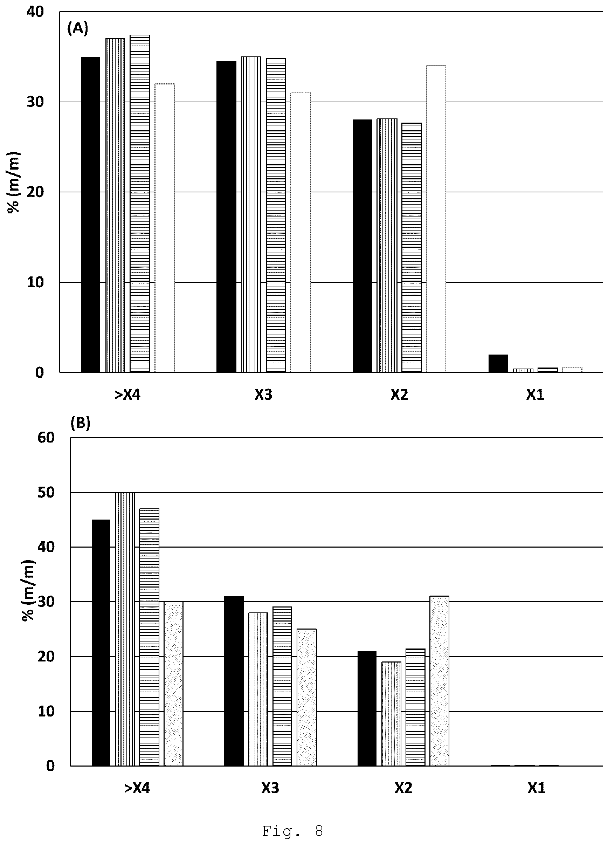

Regarding the evaluation of the prebiotic activity, it was verified that the XOS obtained by the method of the present invention (XOS-BEKP) are resistant to gastrointestinal conditions in all stages of digestion, since the amount of xylose identified in each of the stages was residual (Figure 8).

It is concluded that XOS-BEKP reaches the colon without hydrolysis. In the intestinal phase, there is an increase in the amount of xylobiose in relation to oligosaccharides with a higher degree of polymerization, but the amount of monosaccharides is maintained throughout the digestive phases for XOS-BEKP (Figure 8-A). Since gastrointestinal fluids contain a compound that comes out at the same retention time as xylobiose, and although its amount has been deducted from the final xylobiose value, the determined xylobiose concentration contains an error greater than (standard error shown in the graph) and its variation may not be significant. In the Commercial XOS sample, the percentage increase of an unidentified monosaccharide, which is neither xylose nor arabinose, is also verified in the intestinal phase (Figure 8 -B) .

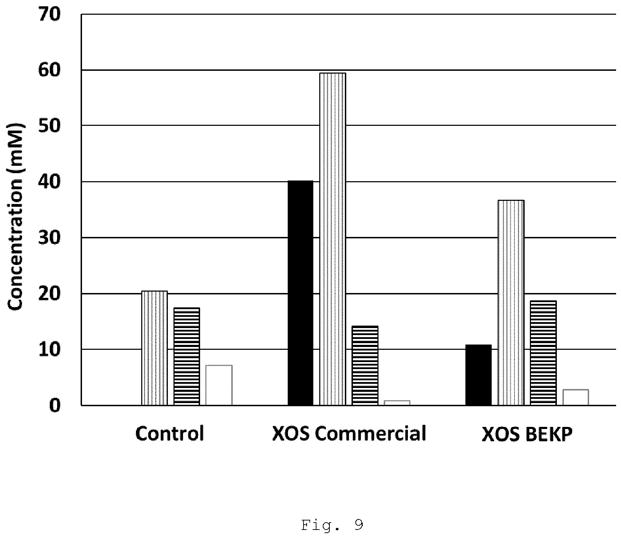

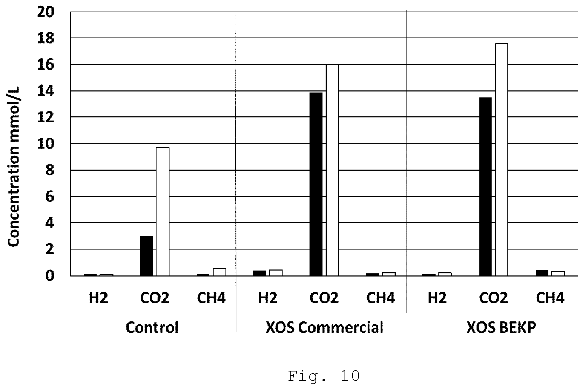

For the two samples of XOS (BEKP and Commercial) changes were observed relative to the control (without prebiotic) regarding: i) increased production of lactate and SCFAs (Figure 9); ii) reduction of ammonia production (crucial factor, since ammonia is carcinogenic and is associated with obesity) (Table 6); iii) increase in $CO_2$ production (inevitable, resulting from fermentative activity) (Figure 10); and iv) pH reduction (resulting from the production of lactate and SCFAs) (Table 6). Thus proving the prebiotic activity of XOS-BEKP.

Table 6. pH and ammonia concentration after 48 h of growth of human fecal inoculum in the absence of prebiotics (Control) or enriched with a solution of XOS-commercial or XOS-BEKP at 10 g/L. Results are the average of two independent fermentations and triplicate analysis of each sample $\pm$ standard deviation.

|  | pH | Ammonia (mg/L) |
|---|---|---|
| Control | 6.93 $\pm$ 0.02 | 189 $\pm$ 7 |
| XOS-Commercial | 3.62 $\pm$ 0.01 | 54 $\pm$ 1 |
| XOS-BEKP | 4.43 $\pm$ 0.01 | 66 $\pm$ 2 |

However, to understand how the XOS affected the microbial population, genetic sequencing of the total fecal inoculum and fecal fermentations (48 h) was carried out. High-throughput sequencing (16S rRNA gene) by Illumina MiSeq technology was performed by RTL Genomics (Lubbock, TX, USA), and samples were analyzed in duplicate. The relative microbial composition (%) after 48 hours of growth is shown in Figure 9 and the respective summary is in Table 7.

Table 7. Relative microbial composition (%) after 48 h of growth of human fecal inoculum in the absence of prebiotics (Control) or enriched with a commercial XOS solution or XOS produced from bleached eucalyptus kraft pulp (XOS-BEKP) at 10 g/L. Results are the mean of two independent fermentations and duplicate analysis of each sample $\pm$ standard deviation.

|  | Control | XOS-Commercial | XOS-BEKP |
|---|---|---|---|
| Bifidobacterium spp. | 4 $\pm$ 1 | 24 $\pm$ 2 | 33 $\pm$ 4 |
| Lactobacillus spp. | <0, 01 | 64 $\pm$ 7 | 10 $\pm$ 6 |
| Clostridium sp. | 8 $\pm$ 1 | 1 $\pm$ 1 | 1 $\pm$ 0,1 |
| Methanobacteriaceae | 2,0 $\pm$ 0,5 | 0,01 $\pm$ 0,01 | 0,05 $\pm$ 0,02 |
| Enterobacteriaceae | 2,0 $\pm$ 0 | 0 $\pm$ 0 | 0,02 $\pm$ 0, 00 |

(continued)

|  | Control | XOS-Commercial | XOS-BEKP |
|---|---|---|---|
| *Proteobacteria* | 10 ± 2 | 0,13 ± 0,10 | 0,95 ± 0, 07 |

By analyzing Table 7, which compares the results for the XOS obtained by the process described here and for the commercially available XOS, an increase in Lactobacillus spp. and Bifidobacterium spp. (beneficial bacteria), as well as the reduction of Clostridium sp., Methanobacteriaceae, Enterobacteriaceae and Proteobacteria (harmful bacteria), from the fecal inoculum after addition of commercial XOS and XOS-BEKP, compared to the control (without prebiotic). Thus, it can be concluded that the XOS produced from bleached Kraft pulp of E. globulus have prebiotic potential with benefits for human health, highlighting a greater development of Bifidobacterium spp in relation to the sample of commercial XOS.

The possibility of incorporating the resulting post-hydrolysis pulp after the production method of the XOS of the present invention, in the normal production of market pulp, that is, pulp to produce paper products, was also evaluated. For this, 2 sets of paper sheets were produced, according to the ISO 5269-1 standard, namely: (1) sheets of paper produced with bleached Kraft pulp of E. globulus on the market; (2) paper sheets produced with 97% (w/w) of normal market pulp and 3% (w/w) of post-hydrolysis pulp resulting after applying the XOS production method as described in this patent application (BEKP-XOS). 3 levels of refining (0, 1500 and 3000 revolutions) were also tested in a PFI refiner according to the ISO 5264-2 standard. These sheets were characterized according to the standards shown in Table 8.

Table 8. Paper properties analyzed and respective associated standards.

| Evaluated property | Norm |
|---|---|
| Drainability | ISO 5267-1 |
| Bulk | ISO 534 |
| Gurley air resistance | ISO 5636-5 |
| Burst index | ISO 2758 |
| Tear index | ISO 1974 |
| Tensile strength | ISO 1924-2 |

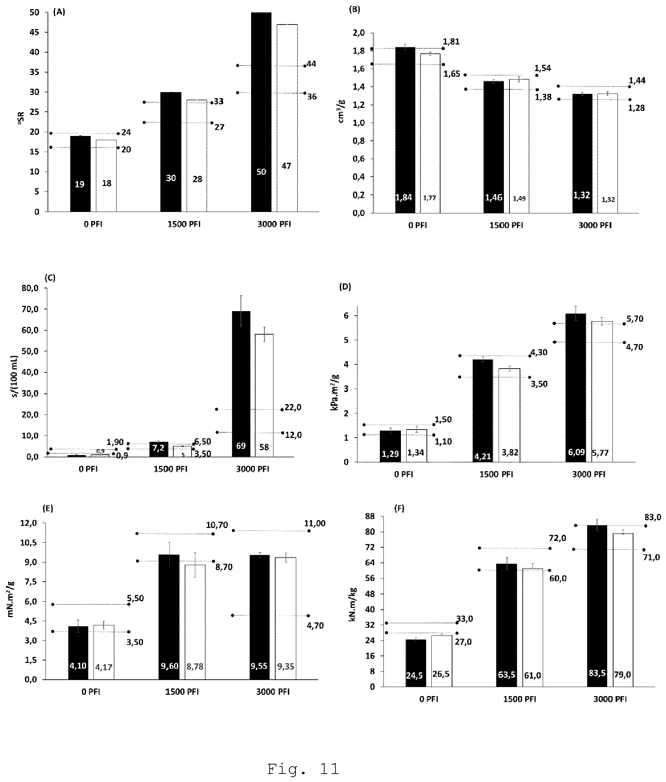

The analyzed papermaking properties are shown in Figure 11 (A to F).

With the incorporation of 3% of the pulp obtained after applying the method described in the present invention, the paper properties are within the expected specifications for a market pulp to produce paper products.

It was then concluded that the incorporation of 3% of the pulp resulting from the XOS production method here described does not cause significant deviations in the final papermaking properties of paper products, making it possible to produce XOS and use the pulp obtained after this process in the normal market pulp production circuit.

Referências

Akpinar, O., Ak, O., Kavas, A., Bakir, U., & Yilmaz, L. (2007). Enzymatic production of xylooligosaccharides from cotton stalks. Journal of Agricultural and Food Chemistry, 55(14), 5544-5551. https://doi.org/10.1021/jf063580d

Bailey, M. J., Biely, P., & Poutanen, K. (1992). Interlaboratory testing of methods for assay of xylanase activity. Journal of Biotechnology, 23(3), 257-270. https://doi.org/10.1016/0168-1656(92)90074-J

Box, G. E. P., & Wilson, K. B. (1951). On the Experimental Attainment of Optimum Conditions. Journal of the Royal Statistical Society: Series B (Methodological), 13(1), 1-45. https://doi.org/10.1111/j.2517-6161.1951.tb00067.x

Cebin, A. V., Ralet, M. C., Vigouroux, J., Karaca, S., Martinic, A., Komes, D., & Bonnin, E. (2021). Valorisation of walnut shell and pea pod as novel sources for the production of xylooligosaccharides. Carbohydrate Polymers, 263. https://doi.org/10.1016/j.carbpol.2021.117932

Chen, X., Cao, X., Sun, S., Yuan, T., Shi, Q., Zheng, L., & Sun, R. (2018). Evaluating the production of monosaccharides and xylooligosaccharides from the pre-hydrolysis liquor of kraft pulping process by acid and enzymatic hydrolysis. Industrial Crops and Products, 124(15), 906-911. https://doi.org/10.1016/j.indcrop.2018.08.071

Cunningham, M., Azcarate-Peril, M. A., Barnard, A., Benoit, V., Grimaldi, R., Guyonnet, D., Holscher, H. D., Hunter, K., Manurung, S., Obis, D., Petrova, M. I., Steinert, R. E., Swanson, K. S., van Sinderen, D., Vulevic, J., & Gibson, G. R. (2021). Shaping the Future of Probiotics and Prebiotics. In Trends in Microbiology (pp. 1-19). https://doi.org/10.1016/j.tim.2021.01.003

EFSA, P. on D. P. N. and A. (NDA). (2018). Safety of xylo-oligosaccharides (XOS) as a novel food pursuant to Regulation (EU) 2015/2283. EFSA Journal. https://doi.org/10.2903/j.efsa.2018.5361

FDA. (2013). GRAS Notice (GRN) No. 458. http://www.fda.gov/Food/IngredientsPackagingLabeling/GRAS/Notice Inventory/default.htm

FDA. (2019). GRAS Notice No. GRN 000816. default.htm

Figueiredo, F. C., Carvalho, A. F. A., Brienzo, M., Campioni, T. S., & de Oliva-Neto, P. (2017). Chemical input reduction in the arabinoxylan and lignocellulose alkaline extraction and xylooligosaccharides production. Bioresource Technology, 228, 164-170. https://doi.org/10.1016/j.biortech.2016.12.097

Gullón, P., Salazar, N., Muñoz, M. J. G., Gueimonde, M., Ruas-Madiedo, P., de los Reyes-Gavilán, C. G., & Parajó, J. C. (2011). Assessment on the fermentability of xylooligosaccharides from rice husks. BioResources, 6(3), 3096-3114. https://doi.org/10.1021/jf800715b

Hakala, T. K., Liitiä, T., & Suurnäkki, A. (2013). Enzyme-aided alkaline extraction of oligosaccharides and polymeric xylan from hardwood kraft pulp. Carbohydrate Polymers, 93(1), 102-108. https://doi.org/10.1016/j.carbpol.2012.05.013

Hong, C., Corbett, D., Venditti, R., Jameel, H., & Park, S. (2019). Xylooligosaccharides as prebiotics from biomass autohydrolyzate. LWT, 111, 703-710. https://doi.org/10.1016/j.lwt.2019.05.098

Hu, J., Zhang, L., Lin, W., Tang, W., Chan, F. K. L., & Ng, S. C. (2021). Review article: Probiotics, prebiotics and dietary approaches during COVID-19 pandemic. In Trends in Food Science and Technology (Vol. 108, pp. 187-196). https://doi.org/10.1016/j.tifs.2020.12.009

Huntley, N. F., & Patience, J. F. (2018). Xylose: Absorption, fermentation, and post-absorptive metabolism in the pig. In Journal of Animal Science and Biotechnology (Vol. 9, Issue 1, pp. 1-9). https://doi.org/10.1186/s40104-017-0226-9

Khaled, J. M. A. (2021). Probiotics, prebiotics, and COVID-19 infection: A review article. In Saudi Journal of Biological Sciences (Vol. 28, Issue 1, pp. 865-869). https://doi.org/10.1016/j.sjbs.2020.11.025

Mano, M. C. R., Neri-Numa, I. A., da Silva, J. B., Paulino, B. N., Pessoa, M. G., & Pastore, G. M. (2018). Oligosaccharide biotechnology: an approach of prebiotic revolution on the industry. Applied Microbiology and Biotechnology, 102(1), 17-37. https://doi.org/10.1007/s00253-017-8564-2

Marques, A. I., Serrano, M. de L., Alves, A. M. B., & de Sousa, A. P. M. (2019). Isolation of xylans from bleached Eucalyptus kraft pulp by antisolvents precipitation. Cellulose, 26(3), 1977-1992. https://doi.org/10.1007/s10570-018-1941-x

Moura, P., Barata, R., Carvalheiro, F., Gírio, F., Loureiro-Dias, M. C., & Esteves, M. P. (2007). In vitro fermentation of xylo-oligosaccharides from corn cobs autohydrolysis by Bifidobacterium and Lactobacillus strains. LWT - Food Science and Technology, 40, 963-972. https://doi.org/10.1016/j.lwt.2006.07.013

Palaniappan, A., Antony, U., & Emmambux, M. N. (2021). Current status of xylooligosaccharides: Production, characterization, health benefits and food application. In Trends in Food Science and Technology (Vol. 111, pp. 506-519). https://doi.org/10.1016/j.tifs.2021.02.047

Palaniappan, A., Balasubramaniam, V. G., & Antony, U. (2017). Prebiotic potential of xylooligosaccharides derived from finger millet seed coat. Food Biotechnology, 31(4), 264-280. https://doi.org/10.1080/08905436.2017.1369433

Park, H. W., Kim, M. J., Seo, S., Yoo, S., & Hong, J. H. (2017). Relative sweetness and sweetness quality of Xylobiose. Food Science and Biotechnology, 26(3), 689-696. https://doi.org/10.1007/s10068-017-0109-z

Pinto, P. C., Evtuguin, D. V., & Neto, C. P. (2005). Structure of hardwood glucuronoxylans: Modifications and impact on pulp retention during wood kraft pulping. Carbohydrate Polymers, 60, 489-497. https://doi.org/10.1016/j.carbpol.2005.03.001

Pollet, A., Delcour, J. A., & Courtin, C. M. (2010). Structural determinants of the substrate specificities of xylanases from different glycoside hydrolase families. Critical Reviews in Biotechnology, 30(3), 176-191. https://doi.org/10.3109/07388551003645599

Samanta, A. K., Jayapal, N., Jayaram, C., Roy, S., Kolte, A. P., Senani, S., & Sridhar, M. (2015). Xylooligosaccharides as prebiotics from agricultural by-products: Production and applications. Bioactive Carbohydrates and Dietary Fibre, 5, 62-71. https://doi.org/10.1016/j.bcdf.2014.12.003

Samanta, A. K., Jayapal, N., Kolte, A. P., Senani, S., Sridhar, M., Dhali, A., Suresh, K. P., Jayaram, C., & Prasad, C. S. (2015). Process for Enzymatic Production of Xylooligosaccharides from the Xylan of Corn Cobs. Journal of Food Processing and Preservation, 39(6), 729-736. https://doi.org/10.1111/jfpp.12282

Samanta, A. K., Jayapal, N., Kolte, A. P., Senani, S., Sridhar, M., Suresh, K. P., & Sampath, K. T. (2012). Enzymatic production of xylooligosaccharides from alkali solubilized xylan of natural grass (Sehima nervosum). Bioresource Technology, 112, 199-205. https://doi.org/10.1016/j.biortech.2012.02.036

Santibáñez, L., Henríquez, C., Corro-Tejeda, R., Bernal, S., Armijo, B., & Salazar, O. (2021). Xylooligosaccharides from lignocellulose biomass: A comprehensive review. In Carbohydrate Polymers (Vol. 251). https://doi.org/10.1016/j.carbpol.2020.117118

Selvendran, R. R., March, J. F., & Ring, S. G. (1979). Determination of aldoses and uronic acid content of vegetable fiber. Analytical Biochemistry, 96(2), 282-292. https://doi.org/10.1016/0003-2697(79)90583-9

Shatalov, A. A., Evtuguin, D. V., & Pascoal Neto, C. (1999). (2-O-$\alpha$-D-Galactopyranosyl-4-O-methyl-$\alpha$-D-glucurono)-D-xylan from Eucalyptus globulus Labill. Carbohydrate Research, 320(1-2), 93-99. https://doi.org/10.1016/S0008-6215(99)00136-6

Singh, R. D., Banerjee, J., Sasmal, S., Muir, J., & Arora, A. (2018). High xylan recovery using two stage alkali pretreatment process from high lignin biomass and its valorisation to xylooligosaccharides of low degree of polymerisation. Bioresource Technology, 256, 110-117. https://doi.org/10.1016/j.biortech.2018.02.009

TAPPI T 412 om-11. (2011). Moisture in Pulp, Paper and Paperboard. TAPPI Press.

Wang, Y., Cao, X., Zhang, R., Xiao, L., Yuan, T., Shi, Q., & Sun, R. (2018). Evaluation of xylooligosaccharide production from residual hemicelluloses of dissolving pulp by acid and enzymatic hydrolysis. RSC Advances, 8, 35211-35217. https://doi.org/10.1039/c8ra07140c

Zheng, C., Lei, Y., Yu, Q., Lui, X., & Huan, K. (2002). Enzymatic hydrolysis of waste sugarcane bagasse in water media. Environmental Technology (United Kingdom), 23(9), 1009-1016. https://doi.org/10.1080/09593332308618349

Minekus, M., Alminger, M., Alvito, P., Ballance, S., Bohn, T., Bourlieu, C. et al. (2014). A standardised static in vitro digestion method suitable for food - an international consensus. Food & Function, 5, 1113-1124.

Krul, E. s. (2019). Calculation of Nitrogen-to-Protein Conversion Factors: A Review with a Focus on Soy Protein. J. Am. Oil. Chem. Soc., 96, 339-364.

## Claims

1. A process for producing xylooligosaccharides comprising the following steps:

   a) enzymatic hydrolysis of a bleached Kraft cellulose pulp with an endoxylanase, in water or in an aqueous medium, in the absence of a buffer solution, at temperatures between 40 °C and 85 °C and for a period of time of up to 8 hours;
   b) separating the aqueous solution of xylooligosaccharides from the post-hydrolysis cellulose pulp resulting from step a);
   c) purifying the aqueous solution of xylooligosaccharides obtained in step b).

2. The process according to the preceding claim wherein the enzymatic hydrolysis of step a) is carried out at temperatures between 60 °C and 85 °C and for a period of time between 4 and 8 hours.

3. The process according to any of the preceding claims, wherein the enzymatic hydrolysis of step a) takes place at temperatures between 75°C and 85°C and for a period of time of 6 hours.

4. The process according to any of the preceding claims, wherein the bleached Kraft cellulose pulp is selected from the group consisting of hardwood, softwood and mixtures thereof.

5. The process according to any of the preceding claims, wherein the bleached Kraft cellulose pulp is from *Eucalyptus globulus.*

6. The process according to any of the preceding claims, wherein the aqueous medium of step a) is a filtrate accompanying a bleached Kraft cellulose pulp.

7. The process according to any one of the preceding claims, wherein the step c) of purifying the aqueous solution of xylooligosaccharides is selected from the group consisting of extraction and precipitation with solvents, adsorption on activated carbon, vacuum filtration and nanofiltration.

8. Xylooligosaccharides obtainable by the process of any one of claims 1 to 7.

9. The xylooligosaccharides according to the previous claim, comprising at least 50% w/w of xylobiose and 40% w/w of xylotriose.

10. The Xylooligosaccharides according to any of claims 8 and 9, comprising at least 0.5% w/w of 4-O-methylglucuronic acid.

11. A post-hydrolysis cellulose pulp obtainable by the process of any of claims 1 to 7.

12. A use of the post-hydrolysis cellulose pulp according to the previous claim, wherein it is integrated into a Kraft pulp production process in an amount of up to 3% w/w.

Fig. 1

Fig. 2

Fig.3

...

Fig.4

Fig. 5

Fig. 6

Fig. 7

Fig. 8

Fig. 9

Fig. 10

Fig. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20190014407 A
- EP 1304412 B1
- US 6942754 B2

**Non-patent literature cited in the description**

- **AKPINAR, O. ; AK, O. ; KAVAS, A. ; BAKIR, U ; YILMAZ, L.** Enzymatic production of xylooligosaccharides from cotton stalks. *Journal of Agricultural and Food Chemistry,* 2007, vol. 55 (14), 5544-5551, https://doi.org/10.1021/jf063580d
- **BAILEY, M. J. ; BIELY, P. ; POUTANEN, K.** Inter-laboratory testing of methods for assay of xylanase activity. *Journal of Biotechnology,* 1992, vol. 23 (3), 257-270, https://doi.org/10.1016/0168-1656(92)90074-J
- **BOX, G. E. P. ; WILSON, K. B.** On the Experimental Attainment of Optimum Conditions. *Journal of the Royal Statistical Society: Series B (Methodological),* 1951, vol. 13 (1), 1-45, https://doi.org/10.1111/j.2517-6161.1951.tb00067.x
- **CHEN, X. ; CAO, X. ; SUN, S. ; YUAN, T. ; SHI, Q. ; ZHENG, L ; SUN, R.** Evaluating the production of monosaccharides and xylooligosaccharides from the pre-hydrolysis liquor of kraft pulping process by acid and enzymatic hydrolysis. *Industrial Crops and Products,* 2018, vol. 124 (15), 906-911, https://doi.org/10.1016/j.indcrop.2018.08.071
- **CUNNINGHAM, M. ; AZCARATE-PERIL, M. A. ; BARNARD, A. ; BENOIT, V. ; GRIMALDI, R. ; GUYONNET, D. ; HOLSCHER, H. D. ; HUNTER, K. ; MANURUNG, S. ; OBIS, D.** Shaping the Future of Probiotics and Prebiotics. *Trends in Microbiology,* 2021, 1-19, https://doi.org/10.1016/j.tim.2021.01.003
- Safety of xylo-oligosaccharides (XOS) as a novel food pursuant to Regulation (EU) 2015/2283. *EFSA Journal,* 2018, https://doi.org/10.2903/j.efsa.2018.5361
- *GRAS Notice (GRN),* 2013, (458, http://www.fda.gov/Food/IngredientsPackagingLabeling/GRAS/Notice Inventory/default.htm
- *GRAS Notice No. GRN 000816,* 2019
- **FIGUEIREDO, F. C. ; CARVALHO, A. F. A. ; BRIENZO, M. ; CAMPIONI, T. S. ; DE OLIVA-NETO, P.** Chemical input reduction in the arabinoxylan and lignocellulose alkaline extraction and xylooligosaccharides production. *Bioresource Technology,* 2017, vol. 228, 164-170, https://doi.org/10.1016/j.biortech.2016.12.097
- **GULLÓN, P. ; SALAZAR, N. ; MUÑOZ, M. J. G. ; GUEIMONDE, M. ; RUAS-MADIEDO, P. ; DE LOS REYES-GAVILÁN, C. G. ; PARAJÓ, J. C.** Assessment on the fermentability of xylooligosaccharides from rice husks. *BioResources,* 2011, vol. 6 (3), 3096-3114, https://doi.org/10.1021/jf800715b
- **HAKALA, T. K. ; LIITIÄ, T. ; SUURNÄKKI, A.** Enzyme-aided alkaline extraction of oligosaccharides and polymeric xylan from hardwood kraft pulp. *Carbohydrate Polymers,* 2013, vol. 93 (1), 102-108, https://doi.org/10.1016/j.carbpol.2012.05.013
- **HONG, C. ; CORBETT, D. ; VENDITTI, R. ; JAMEEL, H. ; PARK, S.** Xylooligosaccharides as prebiotics from biomass autohydrolyzate. *LWT,* 2019, vol. 111, 703-710, https://doi.org/10.1016/j.lwt.2019.05.098
- **HU, J. ; ZHANG, L. ; LIN, W. ; TANG, W. ; CHAN, F. K. L. ; NG, S. C.** Review article: Probiotics, prebiotics and dietary approaches during COVID-19 pandemic. *Trends in Food Science and Technology,* 2021, vol. 108, 187-196, https://doi.org/10.1016/j.tifs.2020.12.009
- **HUNTLEY, N. F. ; PATIENCE, J. F.** Xylose: Absorption, fermentation, and post-absorptive metabolism in the pig. *Journal of Animal Science and Biotechnology,* 2018, vol. 9 (1), 1-9, https://doi.org/10.1186/s40104-017-0226-9
- **KHALED, J. M. A.** Probiotics, prebiotics, and COVID-19 infection: A review article. *Saudi Journal of Biological Sciences,* 2021, vol. 28 (1), 865-869, https://doi.org/10.1016/j.sjbs.2020.11.025

- **MANO, M. C. R. ; NERI-NUMA, I. A. ; DA SILVA, J. B. ; PAULINO, B. N. ; PESSOA, M. G. ; PASTORE, G. M.** Oligosaccharide biotechnology: an approach of prebiotic revolution on the industry. *Applied Microbiology and Biotechnology,* 2018, vol. 102 (1), 17-37, https://doi.org/10.1007/s00253-017-8564-2
- **MARQUES, A. I. ; SERRANO, M. DE L. ; ALVES, A. M. B. ; DE SOUSA, A. P. M.** Isolation of xylans from bleached Eucalyptus kraft pulp by antisolvents precipitation. *Cellulose,* 2019, vol. 26 (3), 1977-1992, https://doi.org/10.1007/s10570-018-1941-x
- **MOURA, P. ; BARATA, R. ; CARVALHEIRO, F. ; GÍRIO, F. ; LOUREIRO-DIAS, M. C. ; ESTEVES, M. P.** In vitro fermentation of xylo-oligosaccharides from corn cobs autohydrolysis by Bifidobacterium and Lactobacillus strains. *LWT - Food Science and Technology,* 2007, vol. 40, 963-972, https://doi.org/10.1016/j.lwt.2006.07.013
- **PALANIAPPAN, A. ; ANTONY, U. ; EMMAMBUX, M. N.** Current status of xylooligosaccharides: Production, characterization, health benefits and food application. *Trends in Food Science and Technology,* 2021, vol. 111, 506-519, https://doi.org/10.1016/j.tifs.2021.02.047
- **PALANIAPPAN, A. ; BALASUBRAMANIAM, V. G. ; ANTONY, U.** Prebiotic potential of xylooligosaccharides derived from finger millet seed coat. *Food Biotechnology,* 2017, vol. 31 (4), 264-280, https://doi.org/10.1080/08905436.2017.1369433
- **PARK, H. W. ; KIM, M. J. ; SEO, S. ; YOO, S. ; HONG, J. H.** Relative sweetness and sweetness quality of Xylobiose. *Food Science and Biotechnology,* 2017, vol. 26 (3), 689-696, https://doi.org/10.1007/s10068-017-0109-z
- **PINTO, P. C. ; EVTUGUIN, D. V. ; NETO, C. P.** Structure of hardwood glucuronoxylans: Modifications and impact on pulp retention during wood kraft pulping. *Carbohydrate Polymers,* 2005, vol. 60, 489-497, https://doi.org/10.1016/j.carbpol.2005.03.001
- **POLLET, A. ; DELCOUR, J. A. ; COURTIN, C. M.** Structural determinants of the substrate specificities of xylanases from different glycoside hydrolase families. *Critical Reviews in Biotechnology,* 2010, vol. 30 (3), 176-191, https://doi.org/10.3109/07388551003645599
- **SAMANTA, A. K. ; JAYAPAL, N. ; JAYARAM, C. ; ROY, S. ; KOLTE, A. P. ; SENANI, S. ; SRIDHAR, M.** Xylooligosaccharides as prebiotics from agricultural by-products: Production and applications. *Bioactive Carbohydrates and Dietary Fibre,* 2015, vol. 5, 62-71, https://doi.org/10.1016/j.bcdf.2014.12.003
- **SAMANTA, A. K. ; JAYAPAL, N. ; KOLTE, A. P. ; SENANI, S. ; SRIDHAR, M. ; DHALI, A. ; SURESH, K. P. ; JAYARAM, C. ; PRASAD, C. S.** Process for Enzymatic Production of Xylooligosaccharides from the Xylan of Corn Cobs. *Journal of Food Processing and Preservation,* 2015, vol. 39 (6), 729-736, https://doi.org/10.1111/jfpp.12282
- **SAMANTA, A. K. ; JAYAPAL, N. ; KOLTE, A. P. ; SENANI, S. ; SRIDHAR, M. ; SURESH, K. P. ; SAMPATH, K. T.** Enzymatic production of xylooligosaccharides from alkali solubilized xylan of natural grass (Sehima nervosum). *Bioresource Technology,* 2012, vol. 112, 199-205, https://doi.org/10.1016/j.biortech.2012.02.036
- **SANTIBÁÑEZ, L. ; HENRÍQUEZ, C. ; CORRO-TEJEDA, R. ; BERNAL, S. ; ARMIJO, B. ; SALAZAR, O.** Xylooligosaccharides from lignocellulose biomass: A comprehensive review. *Carbohydrate Polymers,* 2021, vol. 251, https://doi.org/10.1016/j.carbpol.2020.117118
- **SELVENDRAN, R. R. ; MARCH, J. F. ; RING, S. G.** Determination of aldoses and uronic acid content of vegetable fiber. *Analytical Biochemistry,* 1979, vol. 96 (2), 282-292, https://doi.org/10.1016/0003-2697(79)90583-9
- **SHATALOV, A. A. ; EVTUGUIN, D. V. ; PASCOAL NETO, C.** 2-O-α-D-Galactopyranosyl-4-O-methyl-α-D-glucurono)-D-xylan from Eucalyptus globulus Labill. *Carbohydrate Research,* 1999, vol. 320 (1-2), 93-99, https://doi.org/10.1016/S0008-6215(99)00136-6
- **SINGH, R. D. ; BANERJEE, J. ; SASMAL, S. ; MUIR, J. ; ARORA, A.** High xylan recovery using two stage alkali pre-treatment process from high lignin biomass and its valorisation to xylooligosaccharides of low degree of polymerisation. *Bioresource Technology,* 2018, vol. 256, 110-117, https://doi.org/10.1016/j.biortech.2018.02.009
- TAPPI T 412 om-11. Moisture in Pulp, Paper and Paperboard. TAPPI Press, 2011
- **WANG, Y. ; CAO, X. ; ZHANG, R. ; XIAO, L. ; YUAN, T. ; SHI, Q. ; SUN, R.** Evaluation of xylooligosaccharide production from residual hemicelluloses of dissolving pulp by acid and enzymatic hydrolysis. *RSC Advances,* 2018, vol. 8, 35211-35217, https://doi.org/10.1039/c8ra07140c
- **ZHENG, C. ; LEI, Y. ; YU, Q. ; LUI, X. ; HUAN, K.** Enzymatic hydrolysis of waste sugarcane bagasse in water media. *Environmental Technology (United Kingdom),* 2002, vol. 23 (9), 1009-1016, https://doi.org/10.1080/09593332308618349
- **MINEKUS, M. ; ALMINGER, M. ; ALVITO, P. ; BALLANCE, S. ; BOHN, T. ; BOURLIEU, C. et al.** A standardised static in vitro digestion method suitable for food - an international consensus. *Food & Function,* 2014, vol. 5, 1113-1124

- **KRUL, E. S.** Calculation of Nitrogen-to-Protein Conversion Factors: A Review with a Focus on Soy Protein. *J. Am. Oil. Chem. Soc.,* 2019, vol. 96, 339-364